(19) Europäisches Patentamt
European Patent Office
Office européen des brevets

(11) EP 1 701 158 A1

(12) **EUROPEAN PATENT APPLICATION**

(43) Date of publication:
**13.09.2006 Bulletin 2006/37**

(51) Int Cl.:
***G01N 30/88*** (2006.01)

(21) Application number: **05110145.9**

(22) Date of filing: **28.10.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **10.03.2005 IT MI20050381**

(71) Applicant: **Chimar S.r.l.**
**26013 Crema CR (IT)**

(72) Inventors:
• **Del Re, Attilio Amerigo Maria**
**I- 29010, Alseno (PC) (IT)**
• **Trevisan, Marco**
**I-43039, Salsomaggiore Terme (PR) (IT)**
• **Piacentini, Marco**
**I-26013, Crema (CR) (IT)**

(74) Representative: **Zanoli, Enrico et al**
**Zanoli & Giavarini S.r.l.**
**Via Melchiorre Gioia, 64**
**20125 Milano (IT)**

(54) **Method for the determination of the reducing capacity of products or mixtures containing anti-oxidizing substances.**

(57) Method for the determination of the Reducing Capacity of products or mixtures containing anti-oxidizing substances, in particular food, beverages, cosmetics and similar products, including liquid chromatographic separation HPLC of one or more reducing substances from a sample of the above mentioned food, beverages, cosmetics, herbs and similar products and the following on-line determination of such substances by means of a coulometric detector, comprising the following steps:

a) Extracting a hydrophilic substances containing fraction and a lipophilic substances containing fraction from a sample of the product to be analyzed;
b) Subjecting each of said fractions to liquid chromatographic separation;
c) Determining said hydrophilic and/or lipophilic reducing substances by means of a coulometric detector equipped with an electrode set to a potential between 0 and 1000 mV.

The information relating to the amount of each of said hydrophilic and lipophilic reducing substances is processed to obtain the hydrophilic reducing capacity (HRC), the lipophilic reducing capacity (LRC) and the total reducing capacity (TRC), expressed as Trolog concentration or by means of degrees of a corresponding centesimal scale.

Figure 1

**Description**

The present invention is related to a method for the evaluation of the reducing capacity of products or mixtures containing anti-oxidizing substances, particularly food, cosmetics, herbs and similar products.

It is known that fruit, vegetables and cereals are effective in protecting against various diseases, including some type of cancer and some kind of cardiovascular and cerebrovascular diseases. Such a protection is commonly attributed to the action of natural anti-oxidizing components such as vitamin C and E.

An antioxidant is commonly defined as a substance or compound that, at lower concentration than oxidable substrates, significantly prevents from or retards a pro-oxidant initiated oxidation of the substrate. An antioxidant is also defined as a species reducing a pro-oxidant species that would otherwise damage the substrate.

Antioxidants can be classified into two categories: preventive antioxidants and chain-breaking antioxidants. Preventive antioxidants, such as superoxide dismutase, catalase, peroxidase, transferrin, and metal-chelants, inhibit the formation of reactive oxygen species (ROS). Chain breaking antioxidants are compounds that scavenge oxygen-radicals and thereby break radical chain sequences. They include ascorbate, vitamin E, uric acid, bilirubin, polyphenols, etc.

Several methods have been suggested for the evaluation of the Total Antioxidant Capacity (TAC) and the Total Reducing Capacity (TRC) of food or substances of biological interest.

Different anti-oxidizing substances can be active in vivo through different mechanisms, it is thereby impossible to evaluate the total antioxidant activity of a substance by means of a single method. Some methods can measure antioxidant activity in blocking some radicals (TEAC Assay), other methods can measure the reducing activity related to Fe (III) (FRAP Assay), other methods can measure the antioxidant capacity specifically related to peroxide radicals (ORAC and TRAP Assay).

WO 2004/068140 describes a spectrophotometric method for the determination of the TAC based on the use of saffron-extracted crocine. This method is specifically tuned to the TAC evaluation in biological fluids such as the blood.

Methods have been proposed to investigate the reducing capacity of substances based on voltmetric- or amperometric-electrochemical analysis of component previously separated by means of liquid chromatography. Antioxidants are, indeed, electro-active compounds that may react at an electrode set to a specific potential producing a response-signal proportional to their concentration in the sample. Nevertheless, voltmetric-detecting method can not indicate all the electro-active species of interest, as some of these species release the electrons at a not sufficient speed, while amperometric-detecting method is limited by the fact that only 10 % of the substance reacts at the corresponding electrode.

In J. Agric. Food Chem., 1997, 45, 1787-1796, Guo et al. describe a method for the determination of the total reducing capacity (TRC) that combines a separation technique in liquid chromatography, particularly a HPLC, with an electrochemical detection of the antioxidant previously separated. The electrochemical detection is based on the use of a coulometric detector. Coulometric detectors present a higher sensibility and selectivity than amperometric one; moreover, up to 100 % of the electro-active species reacts at the electrode being oxidized or reduced.

Nevertheless, such a method described by Guo provides only the total reducing capacity of the sample, and not that relative to the single contribute of each anti-oxidizing compound present in the sample itself. Also, such a method can not evaluate the anti-oxidizing capacity of lipophilic anti-oxidizing compounds, among which the vitamins A and E are particularly important: therefore the method described by Guo gives incomplete and not selective results.

On the contrary, it would be desirable to have a method for the evaluation of the total reducing capacity of products or mixtures containing anti-oxidizing substances, particularly food, beverages, cosmetics, herbs and similar products, that can determine the reducing capacity of each single component present in the investigated sample, independently from its chemical-nature.

Moreover, it would be desirable to have a method for the evaluation of the reducing capacity of products or mixtures that allows expressing such a capacity in a clear and easily understandable way, even people lacking a specific scientific knowledge.

A first object of the present invention is therefore to provide a method to determine the reducing capacity of products and mixtures containing anti-oxidizing substances that can provide both the total reducing capacity and the individual reducing capacity of each single anti-oxidizing compound present in the investigated sample that is chromatographically separable.

A second object of the present invention is to provide a method that can indicate both the hydrophilic anti-oxidizing substances and the lipophilic anti-oxidizing substances contributes to the reducing capacity.

A further object of the present invention is to provide a method that can express the total reducing capacity of food, beverages, cosmetics, herbs and similar products in a scale resulting easily understandable even to those people with a little or none specific scientific knowledge.

The objects mentioned above and other objects and advantages of the invention are achieved with a method for the determination of the Reducing Capacity of products or mixtures containing anti-oxidizing substances, comprising a liquid chromatographic separation HPLC of one or more reducing substances from a sample of the above mentioned products or mixtures, and the following on-line determination of such substances by means of a coulometric detector, characterized in that:

- a fraction containing hydrophilic substances and a fraction containing lipophilic substances are extracted from a sample of the product to be analyzed;
- each of said fractions is subjected to liquid chromatographic HPLC separation;
- said reducing substances are determined by means of a coulometric detector equipped with an electrode set to a potential between 0 and 1000 mV.

Natural or synthesized products as food, beverages, cosmetics, herbs and similar products, particularly those derived from fruit and vegetables, are very complex mixtures containing several substances both hydrophilic and lipophilic, including the anti-oxidizing substances. The method according to the present invention is based on the coulometric detection of those electro-active substances separated from the investigated sample by means of liquid chromatography HPLC, and it can detect both hydrophilic and lipophilic electro-active substances.

According to the present invention, from a sample of the products mentioned above to be analyzed, a phase containing anti-oxidizing hydrophilic substances is extracted by means of a hydrophilic solvent, and a phase containing anti-oxidizing lipophilic substances is extracted by means of a lipophilic solvent.

In the phase containing the anti-oxidizing hydrophilic substances the Hydrophilic Reducing Capacity (HRC) is determined. In the phase containing the anti-oxidizing lipophilic substances the Lipophilic Reducing Capacity (LRC) is determined.

The hydrophilic solvent can be chosen among oxygenated and nitrogenous solvent according to the nature of the product to be analyzed. Among oxygenated hydrophilic solvent alcohols, esters, ethers and ketones can be chosen, for example methyl, ethyl, propyl, isopropyl, *n*-butyl, isobutyl and t-butyl alcohols or methyl-formate, propyl-formate, ethyl-formate, methylacetate, methyl-propionate esters or again tetrahydrofuran, or acetone or butanone. On the other hand among nitrogenous hydrophilic solvent suitable one can be chosen among the amides, for example *N,N*-dimehylformamide, or among the nitriles, for example acetonitrile, or again among the heterocyclic solvents, for example pyridine.

The lipophilic solvent can be chosen among hydrocarbons, for example *n*-pentane or *n*-hexane or isooctane or cyclohexane, among aromatic hydrocarbons, for example benzene or toluene, among ethers, for example dimethyl ether or diethyl ether or again diisopropyl ether, and among halogenated hydrocarbons dichloromethane, chloroform, carbon tetrachloride.

The above mentioned extractions can be carried out by using the hydrophilic solvent and the lipophilic solvent separately, or, preferably, by means of a mixture of the hydrophilic and the lipophilic solvent together. In both cases the solvents can be chosen among those mentioned above. If a mixture of solvents is used, preferred mixtures are methanol/dichloromethane, ethyl-acetate/chloroform, acetone/chloroform or acetonitrile/ methylene chloride.

In the case a mixture of solvent is chosen, the ratio between the hydrophilic solvent and the lipophilic solvent is comprised within the range 0.1-9, preferably 0.3-7. More preferably such ratio is about 1.

The liquid phase extracted from the sample is first filtered to separate the solid-residuals and then treated with water in excess to separate the hydrophilic and the lipophilic phases. This treatment is preferably performed in an acid aqueous solution to ensure that all acid lipophilic compounds being undissociated. Acids that fulfil well this task are either organic acids, for example formic acid or acetic acid or propionic acid, or inorganic acids, for example hydrochloric acid, sulphuric acid or phosphoric acid.

The hydrophilic phase undergoes a subsequent extraction with the lipophilic solvent. In the case water/methanol forms the hydrophilic phase, this extraction is performed again with dichloromethane. After this extraction, the sample to be analyzed for the determination of the hydrophilic antioxidants content is drawn from the aqueous solution.

Organic phases collected together undergo a further extraction with water; they are then dried, filtered and treated to evaporate the solvent. The dry residue is then dissolved in toluene and the sample to be analyzed for the evaluation of the lipophilic antioxidants content is drawn from this solution.

Hydrophilic substances- and lipophilic substances-containing samples, obtained from the above described extraction-procedures, undergo a HPLC separation. The chromatographic separation is carried out in a reversed-phase column, preferably a SUPELCOSIL ABZ PLUS (Supelco, Bellefonte, USA) column (25 cm x 4.6 mm i.d.) with a flow rate of 1 ml/min and isocratic elution. Alternatively, it can be used a reversed-phase column filled with a stationary phase chosen among those between $C_1$ and $C_{18}$, preferably among $C_8$ or $C_{18}$, with both polar or non polar substituents, like amine groups. Suitable columns are LUNA C18(2) and LUNA C8(2) (Phenomenex, USA) or DISCOVERY C18 and DISCOVERY C8 (Supelco, Bellefonte, USA) or better DISCOVERY RP AMMIDE C16 (Supelco, Bellefonte, USA). This stationary phase can be either absorbed on or bound to an inert support, preferably having particles with a diameter of between 1 and 10 $\mu$m, more preferably of about 5 $\mu$m.

The mobile phase is a solvent or a mixture of solvents with a suitable polarity according to the sample to be analyzed. The solvent or mixture of solvents can be chosen among those mentioned with reference to the extraction-procedures.

The HRC is preferably determined with a mobile phase formed by a 50/50 v/v mixture of methanol and $NaH_2PO_4$ 0.05M, acidified by means of phosphoric acid to a pH between 2 and 6, preferably between 3 and 4, more preferably to a pH=3.

The LRC is preferably determined with a mobile phase formed by a 65:20:15 v/v/v mixture of acetonitrile/methanol/dichloromethane or, alternatively, by using a solution of 13.4 mM of lithium perchlorate in acetonitrile/ethanol/2-propanol (65:32:3, v/v/v). Injected volumes were 10 $\mu$l.

Among the different methods of electrochemical analysis of substances separated by means of HPLC, the coulometric detection, with its sensitivity and selectivity, fits particularly well to the detection of electro-active substances such as the antioxidants present in the products mentioned above.

The coulometric detector emits a signal that is proportional to the current generated by the reduction or the oxidation of the electro-active substance carried by the eluting fluid at an electrode set to a constant potential. The generated current is proportional to the amount of electro-active substance present in the sample, whereby the concentration of the electro-active substance present in the sample can be determined.

Eukaryotic cells have developed a complex antioxidant network to counteract reactive species that are detrimental. Different redox couples form this network and determine the cellular redox state. The relative amount of reduced and oxidized form of each redox couple may determine changes in cellular redox state by shifting this value towards oxidizing or reducing conditions.

The most important redox couple within the cell is glutathione disulfide/glutathione (GSSG/2GSH) present at a concentration far higher than that of any other redox couple and it is truly the most important cellular redox buffer.

At pH=7, the approximate pH of the cell, the redox potential for the GSSG/2GSH couple is about -240 mV in the so called "biochemical scale", and it is shifted by 420 mV respect to the "electrochemical scale". On the other hand, in the outer layers of the cell membrane, which can be in closer contact with oxygen molecules, a higher redox potential may be present.

In the method according to the present invention, the coulometer electrode potential is set to a value between 0 and 1000 mV, preferably between 120 and 780 mV, more preferably between 380 and 680 mV. The potential of the electrode for the detection of the hydrophilic phase and the lipophilic phase can be the same or different. In a preferred embodiment the potential is set to a value between 380 and 420 mV for the hydrophilic phase detection, and to a value between 600 and 680 mV for the lipophilic phase detection.

A suitable coulometer is the ESA Analytical Coulochem Model 5100 (ESA Inc., Chelmsford, U.S.A.) with analytical cell 5010. With these settings a basically linear response is obtained for a concentration between 5 and 60 $\mu$mol/l for the HRC and 5 and 120 $\mu$mol/l for the LRC. Electronic data acquisition and peak integration are performed using a 4400 Varian Integrator. An alternative procedure to perform the coulometric detection is to progressively investigate the redox activity of the same sample at different redox potentials. In this version of the method of the present invention, the investigated sample flows through eight separated analytical cells each of them set to a specific and progressively higher redox potential. In this way the reducing capacity at different potentials can be investigated with one single injection of the sample to be analyzed. A suitable coulometric detector is the ESA CoulArray® (ESA Inc., Chelmsford, U.S.A.)

The HRC and LRC of each antioxidant present in the sample is given by the peak area of each redox species, and the Total HRC (THRC) and the Total LRC (TLRC) are reckoned as the sum of all the HRC and all the LRC respectively. The Total Reducing Capacity, TRC, is given by the sum of the Total HRC, THRC, and the Total LRC, TLRC. Peaks areas are calibrated by means of the standard antioxidant Trolox. Trolox is an analogue of vitamin E and is widely used as a reference substance because it can be dissolved in an aqueous medium, as the salt, as well as in an organic medium, as the acid.

Ascorbic acid, β-carotene, and lycopene peaks are identified by comparison with chromatograms of standard solutions.

To check out the linearity of response, standard Trolox solutions, at concentrations ranging from 5 to 60 $\mu$mol/l for HRC and from 5 to 120 $\mu$mol/l for LRC, are used.

According to another aspect of the present invention, the TRC is expressed in degrees of a scale characterized by two reference values, 0 (marked as $TRC_0$) and 100 (marked $TRC_{100}$), corresponding respectively to the absence of reducing capacity and to the maximal reducing capacity that may be found in a natural food. Such a maximal reducing capacity $TRC_{100}$ corresponds to 5 $\mu$mol equivalent of Trolox / g. It is then fixed the following linear correlation between Y degrees in the reference scale and X $\mu$mol equivalent of Trolox / g of sample to be investigated:

$$Y \, (°Piacentini) = 20 \cdot X \, (\mu mol \; equivalenti \; di \; Trolox \, /g)$$

The degrees of this centesimal scale are named Piacentini degrees. This scale indicates in a simple and easily understandable way the TRC of a product or mixture, without resorting to the use of complex dimensional units, such as the $\mu$mol equivalent of Trolox / g of sample. This scale turns to be easily usable also by people lacking a scientific knowledge and still interested in being informed about the reducing capacity of a food or beverage, cosmetic, herbs or similar products. The expression of the reducing capacity in Piacentini degrees turns to be particularly suitable to indicate the TRC in the labels of the product sold on the wide market.

The invention will be now described with reference to the following examples that are meant to illustrate and no to limit the possible applications of this invention.

## EXAMPLES 1-7

The reducing capacity of the following beverages bought in a supermarket has been determined:

**Table 1**

| Example | Beverage |
| --- | --- |
| 1 | Juice 100% blond orange |
| 2 | Juice 100% grapes |
| 3 | Juice 100% red orange |
| 4 | Juice 100% pineapple |
| 5 | Tomatoes juice |
| 6 | Multivitamin beverage ACE orange, carrot and lemon based |
| 7 | Multivitamin beverage apple and kiwi based |

All chemicals mentioned in the following were obtained from Carlo Erba Reagenti (Milan, Italy) with the exception of the ascorbate (L-isomer), $\beta$-carotene, glutathione, lycopene, and Trolox that were obtained from Sigma Chemical Co. (St Louis, Missouri, U.S.A.). Water was purified by means of a MilliQ filtration system (Millipore CO, Bredford Massachusetts, U.S.A.).

## A) PREPARATION OF THE FRACTIONS CONTAINING THE HYDROPHILIC AND THE LIPOPHLIC PHASES

Two samples of 20 g each were drawn from each beverage.

Each sample of 20 g was extracted for 30 minutes with 100 ml of a dichloromethane/methanol mixture in the ratio 1/1 v/v. The extract was filtered through a glass microfibre filter (Whatman Intenational, UK) and the filter was washed with 20 ml of dichloromethane. Then 100 ml of water acidified by 5 ml/l of $H_3PO_4$ 84% v/v were added to help separating the phases, one of which contains the lipophilic compounds. If necessary, the emulsion is broken down by adding 30 ml of NaCl saturated solution.

The water-methanol phase was jet extracted with 50 ml of dichloromethane (used for the TRC detection). The cleaned up water-methanol phase was eventually retrieved and its volume was adjusted to 200 ml by the addition of acidified water; the resulting aqueous phase was used to determine the hydrophilic reducing capacity (HRC) and the ascorbate content.

All organic phases, containing lipophilic compounds were washed with distilled water two times, dehydrated with anhydrous sodium sulphate, filtered and evaporated in a Rotavapor® Büchi (Flavil, Switzerland). The dry residue was dissolved in 10 ml of toluene and analyzed by HPLC in order to determine the lipophilic reducing capacity (LRC), the lycopene and $\beta$-carotene contents. All operations were carried out under subdued light and in nitrogen saturated containers.

## B) DETERMINATION OF HRC AND LRC

Each extract was analyzed in duplicate in a high-performance liquid chromatograph system, HPLC, consisting of an HP 1090 Liquid Chromatograph (Hewlett Packard) and an ESA analytical Coulochem model 5100A electrochemical detector. The detector was equipped with a model 5010 analytical cell, and set to a redox potential of + 400 mV. Electronic data acquisition and peak integration were performed using a 4400 Varian Integrator.

Chromatographic separation was performed on a SUPELCOSIL ABZ plus (Supelco,

Bellefonte, USA) column of 25 cm x 4.6 mm i.d.; particles size 5 $\mu$m, with a flow rate of 1 ml/min and isocratic elution.

The HRC was determined using 0.05 M $NaH_2PO_4$ (at pH 3, acidified by $H_3PO_4$)/$CH_3OH$ (50/50 v/v), as a mobile phase. The LRC was determined using 13.4 mM lithium perchlorate in acetonitrile/ethanol/ 2-propanol (65:32:3, v/v/v). Injected volumes were 10 $\mu$l.

The HRC and LRC of each antioxidant present in the sample is given by the peak area of every redox species, accordingly the Total HRC (THRC) and the Total LRC (TLRC) were reckoned as the sum of all the HRC and all the LRC respectively. The Total Reducing Capacity TRC is given as the sum of the THRC and the TLRC. Peaks areas were calibrated by means of the standard antioxidant Trolox. Trolox is an analogue of vitamin E and is widely used as a reference substance because it can be dissolved in an aqueous medium, as the salt, as well as in an organic one, as the acid.

Ascorbic acid, $\beta$-carotene, and lycopene peaks were identified by comparison with chromatograms of standard solutions.

To check out the linearity of response, four concentrations of Trolox, ranging from 5 to 60 $\mu$mol/l for HRC and from 5 to 120 $\mu$mol/l for LRC, were injected 3 times. Limit of quantification (LOQ) was reckoned from 3 injections of serial dilutions of Trolox.

Repeatability was evaluated by injecting 10 times Trolox standard solution (10 $\mu$mol/l).

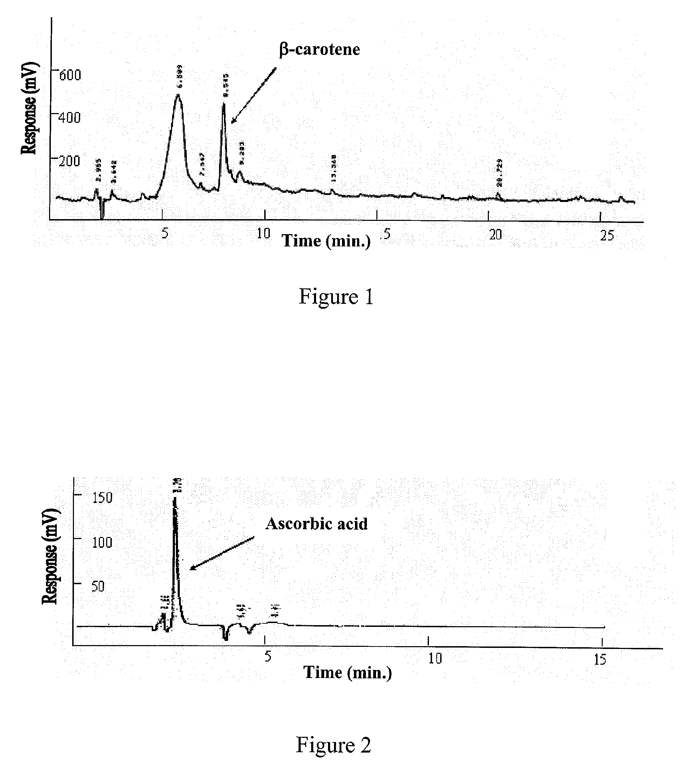

Figure 1 shows the separation of the lipophilic anti-oxidizing substances and Figure 2 shows the separation of the hydrophilic anti-oxidizing substances respectively achieved by means of the method of the present invention applied to the beverage corresponding to the example 6.

Table 2 shows the content of one of the hydrophilic antioxidants (ascorbate) and of two of the lipophilic anti-oxidants ($\beta$-carotene and lycopene) present in the beverages corresponding to the examples listed in Table 1, expressed as $\mu$mol equivalent of Trolox /g.

**Table 2**

| Example | Ascorbate | $\beta$-carotene | Lycopene |
|---|---|---|---|
| 1 | 1.16 | n.d. | n.d. |
| 2 | 1.24 | n.d. | n.d. |
| 3 | 1.85 | n.d. | n.d. |
| 4 | 0.17 | n.d. | n.d. |
| 5 | 0.45 | 0.03 | 1.39 |
| 6 | 2.58 | 0.04 | n.d |
| 7 | 0.51 | 0.10 | n.d. |

Table 3 shows the THRC, the TLRC and the TRC of the beverages corresponding to those examples listed in Table 1, expressed in $\mu$mol equivalent of Trolox /g. The last column of the same Table 3 shows again the TRC of the beverages corresponding to those examples listed in Table 1, now expressed in Piacentini degrees (°Piacentini).

**Table 3**

| Example | THRC | TLRC | TRC | °Piacentini |
|---|---|---|---|---|
| 1 | 1.41 | 0.12 | 1.53 | 10.6 |
| 2 | 1.34 | 0.04 | 1.38 | 27.6 |
| 3 | 2.08 | 0.14 | 2.22 | 44.4 |
| 4 | 0.54 | n.d. | 0.54 | 10.8 |
| 5 | 0.56 | 2.32 | 2.88 | 57.6 |
| 6 | 2.97 | 0.58 | 3.55 | 71.0 |
| 7 | 0.59 | 0.14 | 0.74 | 14.8 |

To check the effectiveness of the method of the present invention, the ascorbate and $\beta$-carotene data reported in Table 2 achieved by means of the method of the present invention have been compared to those content-values given in the label of the corresponding commercial product. Compared data are reported in Table 4. All the data are expressed

in $\mu$mol of substance / g.

**Table 4**

| Example | Content label-guaranteed | | Content determined by the method of the present invention | |
|---|---|---|---|---|
| | Ascorbate | β-carotene | Ascorbate | β-carotene |
| 1 | 0.85 | | 1.48 | |
| 3 | 0.25 | | 1.82 | |
| 4 | 0.25 | | 0.17 | |
| 6 | 0.85 | 0.04 | 4.03 | 0.17 |
| 7 | 0.51 | 0.02 | 0.96 | 0.06 |

As clearly shown in Table 4, these data obtained by means of the method of the present invention give higher values than thee minimum-values guaranteed in the commercial product label. Such a result shows the higher sensitivity and selectivity of the method of the present invention compared to the known methods used to determine the values indicated in the marketed product label.

The following conclusions can be drawn from the results above:

- β-carotene was found in the tomato juice (ex.5), in the multivitamin beverage ACE (ex. 6) and in the multivitamin beverage apple-kiwi (ex. 7), but it was not possible to find it in the orange, grape and pineapple juices, being below the quantification limit (but not always below the detection limit).
- Lycopene was found only in the tomato juice.
- The THRC of the red orange resulted to be higher than that of the blond orange, probably due to the higher ascorbate content registered for the red orange (see Table 2, ex. 1 and 3).
- The highest TLRC registered is that of the tomato juice, while resulted to be not even measurable in the pineapple juice (see Tabel 3).
- The highest TRC turned to be that of the multivitamin beverage ACE and the smallest that of the pineapple juice.

## EXAMPLES 8-9

The reducing capacity of the following food bought in a supermarket was determined:

**Table 5**

| Example | Food |
|---|---|
| 8 | Fresh Tomato |
| 9 | Tomato Sauce (semi-pasteurized) |

All chemicals mentioned in the following were obtained from Carlo Erba Reagenti (Milan, Italy) with the exception of the Trolox that was obtained from Sigma Chemical CO (St Louis,

Missouri, U.S.A.). Water was purified by means of a MilliQ filtration system (Millipore CO, Bredford Massachusetts, U.S.A.).

## A) PREPARATION OF THE FRACTIONS CONTAINING THE HYDROPHILIC PHASE

50 g of fresh samples were analyzed. The samples were chopped by means of a cooled-blender in an inert atmosphere ($N_2$ saturated) to prevent oxygen being introduced in the mixture and in a temperature controlled mode to avoid the temperature to rise.

The sample was weighted directly in a centrifuge can and extracted with 50 ml of double-distilled water acidified by 5 ml/l of $H_3PO_4$ 85% v/v centrifuging at 6000 rpm for 5 minutes. The extract was filtered through a glass microfibre filter (Whatman Intenational, UK): this extraction was repeated twice. The cleaned up phase was eventually retrieved and its volume was adjusted to 200 ml by the addition of the acidified water; the resulting aqueous phase was used to determine the hydrophilic reducing capacity (HRC).

## B) PREPARATION OF THE FRACTIONS CONTAINING THE LIPOPHILIC PHASE

20 g of each sample were drawn and extracted for 30 minutes with 50 ml of methanol and 80 ml of dichloromethane. The extract was filtered through a glass microfibre filter (Whatman Intenational, UK) and the filter was washed with 20 ml of dichloromethane. The solid phase was extracted again for 15 minutes with 30 ml of methanol and 50 ml of dichloromethane, filtered and washed with 20 ml of dichloromethane.

100 ml of double-distilled water were then added to the extract to help separating the phases, one of which contained the lipophilic compounds. The emulsion was broken down by adding 30 ml of NaCl saturated solution. The organic phase was retrieved and the water-methanol phase was again extracted with 50 ml of dichloromethane.

All organic phases, containing the lipophilic compounds, were washed with distilled water two times, dehydrated with sodium anhydrous sulphate, filtered and evaporated in a Rotavapor® Büchi (Flavil, Switzerland). The dry residue was dissolved in 10 ml of ethyl acetate and analyzed by HPLC in order to evaluate the lipophilic reducing capacity (LRC).

All operations were carried out under subdued light and in nitrogen saturated containers.

## C) HRC AND LRC DETERMINATION

Each extract was analyzed in duplicate in a high-performance liquid chromatograph system, HPLC, consisting of an HP 1090 Liquid Chromatograph (Hewlett Packard) and an ESA analytical Coulochem model 5100A electrochemical detector. The detector was equipped with a model 5010 analytical cell, and set to a redox potential of + 400 mV. Electronic data acquisition and peak integration were performed using a 4400 Varian Integrator.

The chromatographic separation was performed on a SUPELCOSIL ABZ plus (Supelco,

Bellefonte, USA) column (25 cm x 4.6 mm i.d.; particles size 5 $\mu$m), with a flow rate of 1 ml/min and isocratic elution.

The HRC was determined using 0.05 M $NaH_2PO_4$ (at pH 3, acidified by $H_3PO_4$)/$CH_3OH$ (50/50 v/v), as a mobile phase. The LRC was determined using 13.4 mM lithium perchlorate in acetonitrile/ethanol/ 2-propanol (65:32:3, v/v/v). Injected volumes were 10 $\mu$l.

The HRC and LRC of each antioxidant present in the sample was given by the peak area of every redox species. Peaks areas were calibrated by means of the standard antioxidant Trolox.

Ascorbic acid, $\beta$-carotene, and lycopene peaks were identified by comparison with chromatograms of standard solutions.

To check out the linearity of response, four concentrations of Trolox, ranging from 5 to 60 $\mu$mol/l for HRC and from 5 to 120 $\mu$mol/l for LRC, were injected 3 times. Limit of quantification (LOQ) was reckoned from 3 injections of serial dilutions of Trolox.

Repeatability was evaluated by injecting 10 times Trolox standard solution (10 $\mu$mol/l).

Table 6 shows the content of one of the hydrophilic antioxidants (ascorbate) and of two of the lipophilic antioxidants ($\beta$-carotene and lycopene) present in the investigated products listed in Table 5, expressed in $\mu$mol equivalent of Trolox /g.

**Table 6**

| Example | Ascorbate | $\beta$-carotene | Lycopene |
|---------|-----------|------------------|----------|
| 8 | 0.687 | 0.005 | 0.904 |
| 9 | 0.687 | 0.011 | 1.255 |

The following conclusions can be drawn from the results above:

- Ascorbate was found in both fresh tomato and semi-pasteurized tomato sauce, in a comparable amount indicating that its concentration is not remarkably affected by the processing of the natural product.
- $\beta$-carotene was found in both the investigated samples in a concentration about double in semi-pasteurized tomato sauce than in fresh tomato, probably due to the lower concentration of water in the sauce compared to the fresh tomato.
- Lycopene was found in both the investigated samples in a concentration 1.4 times higher in the semi-pasteurized tomato sauce than in the fresh product.
- The reducing capacity of the semi-pasteurized tomatoes sauce results to be higher than that of the fresh tomato due to the higher concentration of lipophilic reducing substances.

## EXAMPLES 10-11

The reducing capacity of the following food bought in a supermarket was determined:

**Table 7**

| Example | Food |
|---------|------|
| 10 | Snacks with apricot jam |
| 11 | Toasted slices of bread |

All chemicals mentioned in the following were obtained from Carlo Erba Reagenti (Milan, Italy) with the exception of Trolox that was obtained from Sigma Chemical Co. (St Louis, Missouri, U.S.A.). Water was purified by means of a MilliQ filtration system (Millipore CO, Bredford Massachusetts, U.S.A.).

## A) PREPARATION OF THE FRACTIONS CONTAINING THE HYDROPHILIC PHASE AND THE LIPOPHLIC PHASES

20 g of each sample were drawn and extracted for 30 minutes with 100 ml of a dichloromethane/methanol mixture in the ratio 1/1 v/v. The extract was filtrated through a glass microfibre filter (Whatman Intenational, UK) and the filter was washed with 20 ml of dichloromethane. Then 100 ml of water acidified by 5 ml/l of $H_3PO_4$ 84% v/v were added to help separating the phases, one of which contains the lipophilic compounds. If necessary, the emulsion is broken down by adding 30 ml of NaCl saturated solution.

The water-methanol phase was jet extracted with 50 ml of dichloromethane (used for the TRC detection). The cleaned up water-methanol phase was eventually retrieved and its volume was adjusted to 200 ml by the addition of acidified water; the resulting aqueous phase was used to evaluate hydrophilic reducing capacity (HRC).

All organic phases containing lipophilic compounds were washed with distilled water two times, dehydrated with sodium anhydrous sulphate, filtered and evaporated in a Rotavapor® Büchi (Flavil, Switzerland). The dry residual was dissolved in 10 ml of ethyl acetate and analyzed by HPLC in order to evaluate lipophilic reducing capacity (LRC). All operations were carried out under subdued light and in nitrogen saturated containers.

## B) HRC AND LRC DETERMINATION

Each extract was analyzed in a high-performance liquid chromatograph system, HPLC, consisting in an ESA 582 HPLC system, capable of gradient elution and automatic injections. The column was thermostated by an ESA thermostatic chamber system. The system was connected to a model Coulochem III multielectrode detector equipped with a model 5011 A high sensitivity analytical cell (ESA/Chelmsford, USA), and set to a redox potential of + 400 mV. Electronic data acquisition and peak integration were performed using ESA software.

Chromatographic separation was performed on a SUPELCOSIL ABZ plus (Supelco,

Bellefonte, USA) column (25 cm x 4.6 mm i.d.; particles size 5 $\mu$m), with a flow rate of 1 ml/min and isocratic elution. The phase containing lipophilic reducing substances was also analyzed by setting the coulometric detector electrode to a potential of 660 mV.

The HRC was determined using 0.05 M $NaH_2PO_4$ (at pH 3, acidified by $H_3PO_4$)/$CH_3OH$ (50/50 v/v), as a mobile phase. The LRC was determined using 13.4 mM lithium perchlorate in acetonitrile/ethanol/ 2-propanol (65:32:3, v/v/v). Injected volumes were 10 $\mu$l.

The HRC and LRC of each antioxidant present in the sample was given by the peak area of every redox species. The Total HRC (THRC) and the Total LRC (TLRC) were reckoned as the sum of all the HRC and all the LRC, respectively. The Total Reducing Capacity TRC was given as the sum of the THRC and the TLRC. Peaks areas were calibrated by means of the standard antioxidant Trolox.

To check out the linearity of response, five concentrations of Trolox, ranging from 1.01 to 101 mg/kg for HRC and from 1.44 to 144 mg/kg for LRC, were injected. The limit of quantification (LOQ) was reckoned from 3 injections of serial dilutions of Trolox.

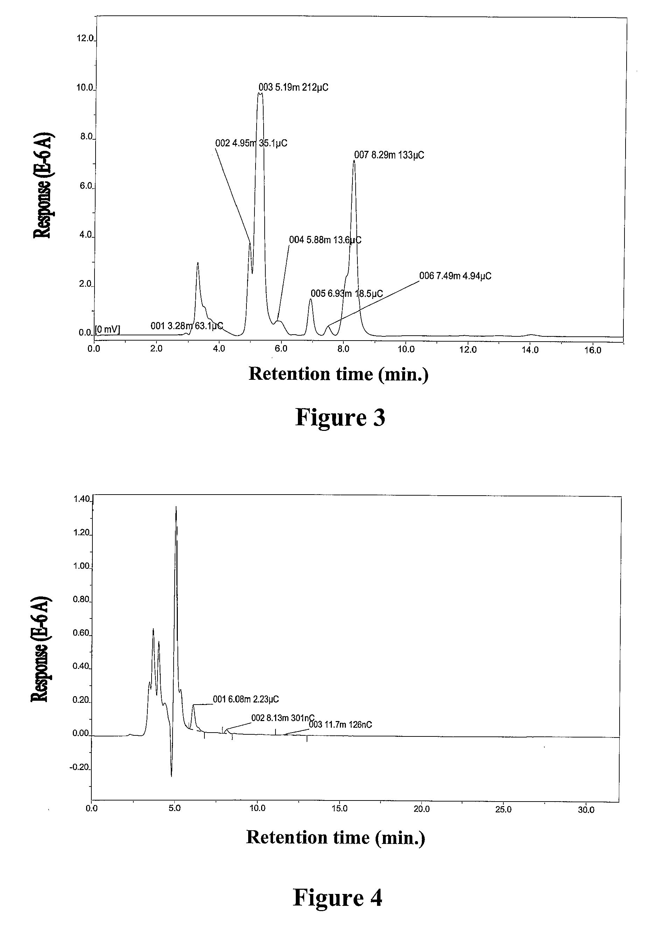

Figure 3 shows the separation of the lipophilic anti-oxidizing substances and Figure 4 shows the separation of the hydrophilic anti-oxidizing substances respectively achieved by means of the method of the present invention when the coulometric detector electrode is set at 400 mV applied to the food corresponding to the example 10.

Table 8 shows the contribution of each single reducing substance present in the products of Table 7 to the Total Lipophilic Reducing Capacity, when the coulometric detector electrode is set to a potential of 400 mV. All values are expressed in $\mu$mol equivalent of Trolox /g. Peaks are labelled progressively according to their retention time. In this way, substances exhibiting the better reducing activity can be easily identified.

**Table 8**

| Example | 10 | 11 |
| --- | --- | --- |
| TLRC (μmol eq.of Trolox /g) | 0.21 | 0.10 |
| Peak n°. 1 | 0.00E+00 | 0.00E+00 |
| Peak n°. 2 | 1.78E-02 | 0.00E+00 |
| Peak n°. 3 | 1.08E-01 | 0.00E+00 |
| Peak n°. 4 | 6.90E-03 | 0.00E+00 |
| Peak n°. 5 | 9.39E-03 | 6.89E-02 |
| Peak n°. 6 | 2.5 1 E-03 | 1.69E-03 |
| Peak n°. 7 | 6.75E-02 | 1.46E-03 |
| Peak n°. 8 | 0.00E+00 | 6.37E-03 |
| Peak n°.9 | 0.00E+00 | 2.15E-03 |
| Peak n°. 10 | 0.00E+00 | 3.01E-03 |
| Peak n°. 11 | 0.00E+00 | 1.61E-02 |
| Peak n°. 12 | 0.00E+00 | 1.40E-03 |
| Peak n°. 13 | 0.00E+00 | 1.80E-03 |
| Peak n°. 14 | 0.00E+00 | 3.79E-04 |
| Peak n°. 15 | 0.00E+00 | 5.86E-04 |
| Peak n°. 16 | 0.00E+00 | 0.00E+00 |
| Peak n°. 17 | 0.00E+00 | 0.00E+00 |

Table 9 shows the contribution of each reducing substance present in the products of Table 7 to the Total Hydrophilic Reducing Capacity, expressed in μmol equivalent of Trolox /g. Peaks are labeled progressively according to their retention time. In this way, substances exhibiting the best reducing activity can be easily identified.

**Table 9**

| Example | 10 | 11 |
| --- | --- | --- |
| TLRC (μmol eq.of Trolox /g) | 0.03 | 0.00 |
| Peak n°.1 | 2.56E-02 | 4.93E-04 |
| Peak n°.2 | 3.45E-03 | 6.65E-05 |
| Peak n°.3 | 1.44E-03 | 2.79E-05 |

Table 10 shows the THRC, the TLRC and the TRC of the foods corresponding to those examples listed in Table 7, expressed in μmol equivalent of Trolox /g. The TRC is expressed also in Piacentini degrees (°Piacentini).

**Table 10**

| Example | THRC400 mV | TLRC400 mV | TRC | °Piacentini |
| --- | --- | --- | --- | --- |
| 10 | 0.03 | 0.21 | 0.24 | 4.8 |
| 11 | 0.00 | 0.10 | 0.10 | 2 |

Table 11 shows the THRC, the TLRC and the TRC of the foods corresponding to those examples listed in Table 7, expressed in μmol equivalent of Trolox /g. In this case, the coulometric detector electrode was set to a potential of 660 mV for the analysis of the phase containing the lipophilic reducing substances. The table shows also the TRC expressed in Piacentini degrees (°Piacentini).

**Table 11**

| Example | THRC 400 mV | TLRC 660 mV | TRC | °Piacentini |
|---|---|---|---|---|
| 10 | 0.03 | 0.23 | 0.26 | 5.2 |
| 11 | 0.00 | 0.11 | 0.11 | 2.2 |

The following conclusions can be drawn from the results above:

- The snack with apricot jam presents both a better THRC and TLRC.
- The snack with apricot jam clearly shows a better TRC, which is likely due to the presence of the fruit-based filling.
- The more active lipophilic reducing substances were those corresponding respectively to peaks number 2,3 and 7 in the example number 1 and to peaks number 5 and 11 in the example number 2.
- The more active hydrophilic reducing substances were those corresponding to peak number 1 in the example 10, while all the other hydrophilic reducing substances showed a very poor reducing activity at 400 mV both, in the food corresponding to the example 10 and 11.
- The direct comparison of the analyses of the lipophilic phase respectively at 400 and 660 mV showed that the large majority of the lipophilic reducing substances contained in the two foods corresponding to the examples listed in Table 7 are active already at 400 mV.

EXAMPLES 12-14

The reducing capacity of the following cosmetics bought in a beauty shop was determined:

**Table 12**

| Example | Cosmetic |
|---|---|
| 12 | Red lipstick |
| 13 | Pink lipstick |
| 14 | Brown lipstick |

All chemicals mentioned in the following were obtained from Carlo Erba Reagenti (Milan, Italy) with the exception of the Trolox that was obtained from Sigma Chemical Co. (St Louis, Missouri, U.S.A.). Water was purified by means of a MilliQ filtration system (Millipore CO, Bredford Massachusetts, U.S.A.).

A) PREPARATION OF THE FRACTIONS CONTAINING THE HYDROPHILIC PHASE AND THE LIPOPHILIC PHASE

The experimental procedures concerning the preparation of the fractions containing the hydrophilic and lipophilic phases were the same of Examples 10-11.

B) HRC AND LRC DETERMINATION

Each extract was analyzed in a high-performance liquid chromatograph system, HPLC, consisting in a ESA 582 HPLC system, capable of gradient elution and automatic injections.
The column was thermostated by a ESA thermostatic chamber system. The system was connected to a model Coulochem III multi-electrode detector equipped with a model 5011 A high sensitivity analytical cell (ESA/Chelmsford, USA), and set to a redox potential of + 400 mV. Electronic data acquisition and peak integration were performed using ESA software.
Chromatographic separation was performed on a SUPELCOSIL ABZ plus (Supelco, Bellefonte, USA) column (25 cm x 4.6 mm i.d.; particles size, 5 $\mu$m), with a flow rate of 1 ml/min and isocratic elution.
The HRC was assessed using 0.05 M $NaH_2PO_4$ (at pH 3, acidified by $H_3PO_4$)/$CH_3OH$ (50/50 v/v), as a mobile phase. The LRC was assessed using 13.4 mM lithium perchlorate in acetonitrile/ethanol/ 2-propanol (65:32:3, v/v/v). Injected volumes were 10 $\mu$l.
The HRC and LRC of each antioxidant present in the sample was given by the peak area of every redox species, accordingly the Total HRC (THRC) and the Total LRC (TLRC) were reckoned as the sum of all the HRC and all the LRC respectively. The Total Reducing Capacity TRC was given as the sum of the THRC and the TLRC. Peaks areas were calibrated by means of the standard antioxidant Trolox. Trolox is an analogue of vitamin E and is widely used as a reference substance because it can be dissolved in an aqueous medium, as the salt, as well as in an organic one, as the acid.

To check out the linearity of response, five concentrations of Trolox, ranging from 5,75 to 575 $\mu$mol/l for HRC and from 4 to 400 $\mu$mol/l for LRC, were injected.

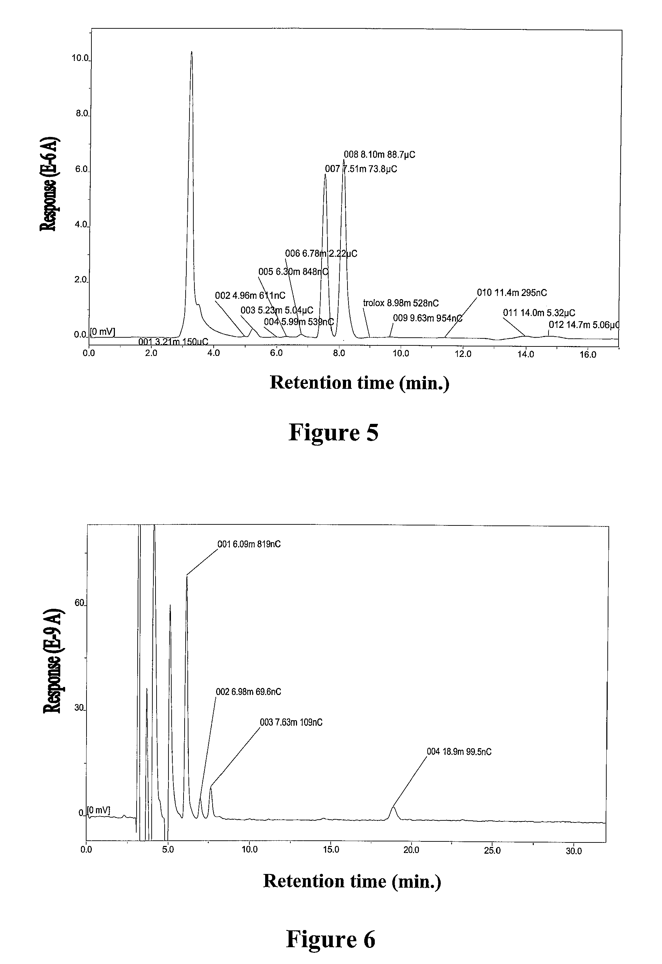

Figure 5 shows the separation of the lipophilic anti-oxidizing substances and Figure 6 shows the separation of the hydrophilic anti-oxidizing substances respectively achieved by means of the method of the present invention when the coulometric detector electrode is set at 400 mV applied to the cosmetic corresponding to the example 12.

Table 13

| Example | 12 | 13 | 14 |
| --- | --- | --- | --- |
| TLRC ($\mu$mol eq.of Trolox /g) | 0.24 | 0.17 | 0.28 |
| Peak n°. 1 | 0.00E+00 | 0.00E+00 | 0.00E+00 |
| Peak n°.2 | 7.87E-04 | 0.00E+00 | 0.00E+00 |
| Peak n°.3 3 | 6.49E-03 | 1.57E-03 | 0.00E+00 |
| Peak n°.4 | 6.94E-04 | 3.03E-03 | 3.37E-03 |
| Peak n°.5 | 1.09E-03 | 3.17E-03 | 4.04E-03 |
| Peak n°.6 | 2.86E-03 | 2.26E-03 | 2.00E-03 |
| Peak n°.7 | 9.50E-02 | 7.06E-02 | 2.08E-03 |
| Peak n°.8 | 1.14E-01 | 7.94E-02 | 4.54E-03 |
| Peak n°.9 | 6.80E-04 | 1.25E-03 | 1.10E-01 |
| Peak n°.10 | 1.23E-03 | 6.95E-04 | 1.38E-01 |
| Peak n°.11 | 3.80E-04 | 1.41E-03 | 1.26E-03 |
| Peak n°.12 | 6.85E-03 | 1.56E-03 | 1.88E-03 |
| Peak n°. 13 | 6.52E-03 | 6.65E-04 | 5.61E-04 |
| Peak n°. 14 | | | 8.69E-04 |
| Peak n°. 15 | | | 1.49E-03 |
| Peak n°. 16 | | | 4.09E-03 |
| Peak n°. 17 | | | 5.66E-03 |

Table 13 shows the contribution of each reducing substance of examples 12-14 to the Total

Lipophilic Reducing Capacity, expressed in $\mu$mol equivalent of Trolox /g. Peaks are labeled progressively according to their retention time. In this way, substances exhibiting the better reducing activity could be easily identified. Corresponding analysis for the Total Hydrophilic Reducing Capacity is not presented here for sake of brevity, especially given the poor THRC profile evidenced by these products.

Table 14 shows the THRC, the TLRC and the TRC of the cosmetic products corresponding to the examples of Table 12, expressed in $\mu$mol equivalent of Trolox /g. The last column of Table 14 shows again the TRC of the cosmetics of the examples of Table 12 expressed in Piacentini degrees (°Piacentini).

Table 14

| Example | THRC 400 mV | TLRC 400 mV | TRC | Piacentini |
| --- | --- | --- | --- | --- |
| 12 | 0.01 | 0.24 | 0.25 | 5 |
| 13 | 0.01 | 0.17 | 0.18 | 3.6 |
| 14 | 0.01 | 0.28 | 0.29 | 5.8 |

The following conclusions can be drawn from the results achieved in the presented investigation:

- The hydrophilic reducing capacity evidenced was very poor for all the three samples, in line with the nature of the cosmetic product.
- A major contribution to the TRC comes from the lipophilic reducing substances.
- The Brown lipstick, which has the higher concentration of $Fe^{2+}$ based pigment, showed the higher TLRC.
- Despite the amount of anti-oxidizing agent introduced by the producer was absolutely the same in all three of the lipsticks, the Brown lipstick showed the higher TRC and the Pink lipstick the lower one.
- Total Reducing Capacity were independent from the specific addition of known anti-oxidizing components, all the ingredients contributed to define the TRC of the final product. All those components exhibiting a redox activity in a range of potential of biological interest were detected and their contribution to the anti-oxidizing capacity of the final product was evaluated.

## EXAMPLE 15

The reducing capacity of the following cosmetic product bought in a beauty shop has been investigated:

**Table 15**

| Example | Cosmetic |
|---------|----------|
| 15 | lipstick |

All chemicals mentioned in the following were obtained from Carlo Erba Reagenti (Milan, Italy) with the exception of Trolox, that was obtained from Sigma Chemical CO (St Louis, Missouri, U.S.A.). Water was purified by means of a MilliQ filtration system (Millipore CO, Bredford Massachusetts, U.S.A.).

A) <u>PREPARATION OF THE FRACTIONS CONTAINING THE HYDROPHILIC PHASE AND THE LIPOPHLIC PHASE</u>

The experimental procedures applied in this example on the preparation of the fractions containing the hydrophilic and lipophilic phases were the same described in connection with Examples 12-14.

B) <u>HRC AND LRC DETERMINATION</u>

Each extract was analyzed in a high-performance liquid chromatograph system HPLC, consisting in a ESA 582 HPLC system, capable of gradient elution and automatic injections.

The column was thermostated by an ESA thermostatic chamber system. The system was connected to a model CoulArray (ESA Inc. Chelmsford, Ma. USA), equipped with 8 electrodes. The detection system consisted of two cell packs in series, each containing four porous graphite working electrodes with associated palladium reference electrodes and platinum counter electrodes. The cells potentials were set as follows: 0/120/400/600/660/720/780/0 mV. Electronic data acquisition and peak integration were performed using CoulArray software.

Chromatographic separation was performed on a SUPELCOSIL ABZ plus (Supelco, Bellefonte, USA) column (25 cm x 4.6 mm i.d.; particles size, 5 $\mu$m), with a flow rate of 1 ml/min and isocratic elution.

The HRC was determined using 0.05 M $NaH_2PO_4$ (at pH 3, acidified by $H_3PO_4$)/$CH_3OH$ (50/50 v/v), as a mobile phase. The LRC was determined using 13.4 mM lithium perchlorate in acetonitrile/ethanol/ 2-propanol (65:32:3, v/v/v). Injected volumes were 10 $\mu$l.

The HRC and LRC of each antioxidant present in the sample is given by the peak area of every redox species, accordingly the Total HRC (THRC) and the Total LRC (TLRC) were reckoned as the sum of all the HRC and all the LRC respectively. The Total Reducing Capacity TRC is given as the sum of the THRC and the TLRC. Peaks areas were calibrated by means of the standard antioxidant Trolox.

To check out the linearity of response, five concentrations of Trolox, ranging from 5,75 to 575 $\mu$mol/l for HRC and from 4 to 400 $\mu$mol/l for LRC, were injected.

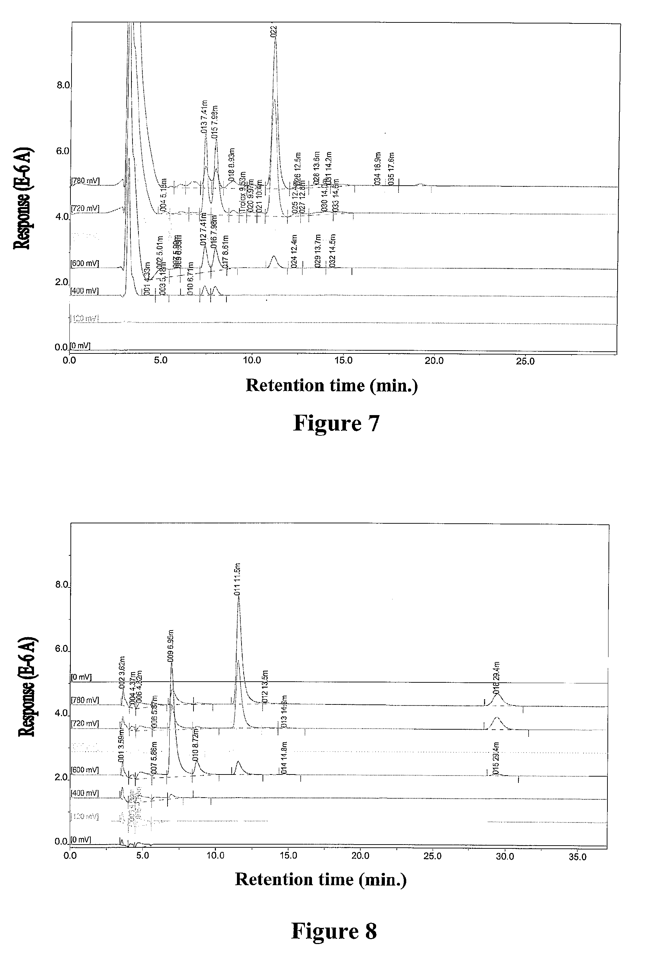

Figure 7 shows the separation of the lipophilic anti-oxidizing substances and Figure 8 shows the separation of the hydrophilic anti-oxidizing substances achieved by an analysis making use of the ColuArray detector at different potentials of the cosmetic products of example 15.

The different redox activity of substances at different potentials is clearly appreciable. In turn, different reducing capacity is exhibited relatively to the operating conditions.

Table 16 shows the contribution to the Total Lipophilic Reducing Capacity of each reducing substance present in the cosmetic products of example number 15 at progressively higher potentials. All data are expressed in $\mu$C. Peaks are labeled progressively according to their retention time. In this way, the behavior of reducing substances can be investigated through different potentials to determine the best reducing substance at each potential.

**Table 16**

| Potential (mV) → | 120 | 400 | 600 | 660 | 720 | 780 | 0 |
|---|---|---|---|---|---|---|---|
| Peak n° | | | | | | | |
| 1 | | 0.254 | | | | | |
| 2 | | | 12.4 | | | | |
| 3 | | 0.282 | | | | | |
| 4 | | | | | 1.23 | | |
| 5 | | | | | | 0.673 | 6.85 |
| 6 | | | | 0.233 | | | |
| 7 | | | 8.87 | | | | |
| 8 | | | | | 2.75 | 2.76 | 2.54 |
| 9 | | | 12.7 | | | | |
| 10 | | 0.541 | | | | | |
| 11 | | | | | 2.1 | 6.9 | 16 |
| 12 | | 4.4 | 14.8 | 2.73 | | | |
| 13 | | | | 2.73 | 37.7 | 13.2 | |
| 14 | | | | | | | 49.7 |
| 15 | | | | 2.73 | 43.3 | 10.3 | |
| 16 | | 4.57 | 13.7 | 2.73 | | | |
| 17 | | | 0.638 | | | | |
| 18 | | | | 0.219 | 3.9 | 10 | |
| 19 | | | | | 2.16 | | |
| 20 | | | | | | | 51.8 |
| 21 | | | | | 3.71 | 3.93 | |
| 22 | | | | | 2.93 | 2.73 | |
| 23 | | | 8.22 | 8 | 80.6 | 99.5 | 7.64 |
| 24 | | | | | | | 9.54 |
| 25 | | | 0.169 | | | | |
| 26 | | | | | 2.27 | | |
| 27 | | | | | | 4.54 | |
| 28 | | | | | 1.93 | | |
| 29 | | | | | | 5.36 | |
| 30 | | | 0.902 | | | | |
| 31 | | | | | 5.98 | | |
| 32 | | | | | | 3.2 | |
| 33 | | | 1.05 | | | | |
| 34 | | | | | 2.32 | | |
| 35 | | | | | | 0.106 | |
| 36 | | | | | | 0.133 | |

(continued)

| Potential (mV) → | 120 | 400 | 600 | 660 | 720 | 780 | 0 |
|---|---|---|---|---|---|---|---|
| 37 | | | | | | 1.29 | 2.07 |

Table 17 shows the THRC, the TLRC and the TRC of the cosmetic product of example 15, expressed in μmol equivalent of Trolox /g, when the electrode potential is set to 720 mV.

**Table 17**

| Example | THRC 720 mV | TLRC 720 mV | TRC |
|---|---|---|---|
| 15 | 1.09 | 1.49 | 2.58 |

The following conclusions can be drawn from the results achieved in the presented investigation:

- Different substances contributed to the TRC at different potential.
- The highest contribute to the TLRC was given by substances corresponding to peaks n° 12 and 16 at 600 mV, to peaks n°13, 15 and 23 at 720 mV, to peak n° 23 at 780 mV and to peak n° 14 at 1.0 V.
- The highest contribute to the THRC was given by substances corresponding to peak 5 at 400, to peaks n° 5, 9 and 10 at 600 mV, to peaks n° 9, 11 and 23 at 660 mV, to peak n° 9, 11 and 16 at 720 mV and 780 mV.

As it appears from the description above, the method of the present invention is particularly advantageous to determine the reducing capacity of food, beverages, cosmetics, herbs and similar products, even if they are of a very complex nature.

- Hydrophilic and Lipophilic substances are extracted, separated and investigated starting from the same one sample;
- All the electro-active components are determined, even if of unknown nature;
- The method of the invention is a direct method, therefore no intermediate reactions or reactants are used;
- The method of the invention is an absolute method, as it determines directly the property of interest;
- The method of the invention is a specific method, as only electro-active reducing components are detected;
- The reducing capacity is determined after the chromatographic separation, it is therefore possible to identify all the anti-oxidizing substances of which a standard sample is known;
- The method of the invention is not a colorimetric method, therefore no interferences occur when a coloured sample is analyzed.

**Claims**

1. Method for the determination of the Reducing Capacity of products or mixtures containing anti-oxidizing substances, comprising a liquid chromatographic separation HPLC of one or more reducing substances from a sample of said products or mixtures, and an on-line determination of said substances by means of a coulometric detector, **characterized by** comprising the following steps:

   a. Extracting a fraction containing hydrophilic substances and a fraction containing lipophilic substances from a sample of the product to be analyzed;
   b. Subjecting each of said fractions to liquid chromatographic HPLC separation;
   c. Determining the amount of said hydrophilic and/or lipophilic reducing substances by means of a coulometric detector equipped with an electrode set to a potential within the range of from 0 to 1000 mV.

2. Method according to claim 1, **characterized by** the fact that the information relative to the amount of each of said hydrophilic and lipophilic reducing substances is processed and expressed as concentration of a reference-standard reducing substance to obtain a hydrophilic reducing capacity (HRC) and a lipophilic reducing capacity (LRC), respectively.

3. Method according to claim 2, **characterized in that** said hydrophilic reducing capacities (HRC) and said lipophilic reducing capacities (LRC) are added up to obtain the total hydrophilic reducing capacity (THRC) and the total lipophilic reducing capacity (TLRC), respectively.

4. Method according to claim 3, **characterized in that** said total hydrophilic reducing capacity (THRC) and said total lipophilic reducing capacity (TLRC) are added up to obtain the total reducing capacity (TRC).

5. Method according to any of the claims from 2 to 4, **characterized in that** said reference-standard reducing substance is Trolox.

6. Method according to claim 1, **characterized in that** said fraction containing hydrophilic reducing substances is extracted by means of a hydrophilic solvent.

7. Method according to claim 6, **characterized in that** said hydrophilic solvent is selected from the groups consisting of alcohols, esters, ethers, ketones, amides, nitriles, heterocyclic compounds.

8. Method according to claim 7, **characterized in that** said hydrophilic solvent is methanol or ethyl acetate.

9. Method according to claim 1, **characterized in that** said fraction containing lipophilic reducing substances is extracted by means of a lipophilic solvent.

10. Method according to claim 9, **characterized in that** said lipophilic solvent is selected from the groups consisting of aliphatic hydrocarbons, aromatic hydrocarbons, halogenated hydrocarbons, ethers, carbonium tetrachloride.

11. Method according to claim 10, **characterized in that** said lipophilic solvent is dichloromethane or chloroform.

12. Method according to claim 1, **characterized in that** said fraction containing hydrophilic reducing substances and said fraction containing lipophilic reducing substances are extracted from the same sample of product or mixture to be analyzed.

13. Method according to claim 11 **characterized in that** said extraction of said hydrophilic and lipophilic reducing substances is carried out by means of a mixture of hydrophilic and lipophilic solvents.

14. Method according to claim 13, **characterized in that** said mixture of solvents is selected from the group consisting of methanol/ dichloromethane, ethyl-acetate/chloroform, acetone/chloroform, acetonitrile/methylene chloride.

15. Method according to claim 13, **characterized in that** the volume ratio between said hydrophilic solvent and said lipophilic solvent is between 0.1-9.

16. Method according to claim 15, **characterized in that** the volume ratio between said hydrophilic solvent and said lipophilic solvent is between 0.3-7.

17. Method according to claim 16, **characterized in that** the volume ratio between said hydrophilic solvent and said lipophilic solvent is about 1.

18. Method according to claim 13, **characterized in that** the liquid phase extracted from said sample is treated with water to separate the lipophilic phase from the hydrophilic phase.

19. Method according to claim 18, **characterized in that** said treatment is performed by means of an aqueous solution of an acid.

20. Method according to claim 19, **characterized in that** above said acid is selected from the group consisting of formic acid, acetic acid, propionic acid, hydrochloric acid, sulphuric acid, phosphoric acid.

21. Method according to claim 1, **characterized in that** said liquid chromatography HPLC separation of one or more reducing substances is performed by means of a column that works in reversed-phase filled with a phase within the range $C_8$ - $C_{18}$.

22. Method according to claim 1, **characterized in that** said electrode of said coulometric detector is set to a potential between 120 and 780 mV.

23. Method according to claim 22, **characterized in that** said electrode of said coulometric detector is set to a potential between 380 and 680 mV.

24. Method according to any of claim 1, 22 or 23 **characterized in that** said potential is the same for the detection of the hydrophilic substances and for the detection of the lipophilic substances.

25. Method according to any of claim 1, 22 or 23 **characterized in that** the potential for the detection of said hydrophilic substances is different from the potential for the detection of said lipophilic substances.

26. Method according to claim 25, **characterized in that** the potential for the detection of said hydrophilic substances is set between 380 and 420 mV, and the potential for the detection of said lipophilic substances is set between 600 and 680 mV.

27. Method according to claim 5, **characterized in that** said total reducing capacity (TRC) expressed as a concentration of a reference-standard reducing substance is converted in degrees of a scale in which the 0 corresponds to the absence of reducing capacity and the 100 corresponds to the reducing capacity of 5 $\mu$mol equivalents of Trolox.

28. Method according to any of the preceding claims, **characterized in that** said products or mixtures containing anti-oxidizing substances are selected from the group consisting of food, beverage, cosmetic products, herbs or mixtures thereof.

Figure 1

Figure 2

**Figure 3**

**Figure 4**

**Figure 5**

**Figure 6**

Figure 7

Figure 8

EP 1 701 158 A1

**European Patent Office**

# EUROPEAN SEARCH REPORT

Application Number

EP 05 11 0145

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 020 205 A (VOJDANI ET AL) 1 February 2000 (2000-02-01) * abstract; figures 1-5 * * column 2, lines 30-50 * * column 4, line 28 - column 5, line 15 * | 1-28 | G01N30/88 |
| D,Y | GUO CHANGJIANG ET AL: "High-performance liquid chromatography coupled with coulometric array detection of electroactive components in fruits and vegetables: Relationship to oxygen radical absorbance capacity" JOURNAL OF AGRICULTURAL AND FOOD CHEMISTRY, vol. 45, no. 5, 1997, pages 1787-1796, XP002369572 ISSN: 0021-8561 * abstract; figure 2; table 4 * * page 1788, left-hand column, last paragraph - page 1789, left-hand column, paragraph 1 * * page 1792, left-hand column, last paragraph - right-hand column, paragraph 1; table 5 * * page 1795, left-hand column, lines 1-14 * | 1-28 | |
| Y | GAO XIANGQUN ET AL: "Changes in antioxidant effects and their relationship to phytonutrients in fruits of sea buckthorn (Hippophae rhamnoides L.) during maturation" JOURNAL OF AGRICULTURAL AND FOOD CHEMISTRY, vol. 48, no. 5, May 2000 (2000-05), pages 1485-1490, XP002369573 ISSN: 0021-8561 * abstract; figure 1 * | 1-28 | **TECHNICAL FIELDS SEARCHED (IPC)** G01N |

-/--

The present search report has been drawn up for all claims

| Place of search | Date of completion of the search | Examiner |
|---|---|---|
| Munich | 24 February 2006 | Hanisch, C |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

22

**European Patent Office**

**EUROPEAN SEARCH REPORT**

Application Number

EP 05 11 0145

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TAKEDA H ET AL: "Simultaneous determination of alpha-tocopherol and alpha-tocopherolquinone by high-performance liquid chromatography and coulometric detection in the redox mode" JOURNAL OF CHROMATOGRAPHY A, ELSEVIER, AMSTERDAM, NL, vol. 722, no. 1, 26 January 1996 (1996-01-26), pages 287-294, XP004038291 ISSN: 0021-9673 * abstract * * page 290, left-hand column, lines 27-35 * | 1-28 | |

----- 

**TECHNICAL FIELDS SEARCHED (IPC)**

The present search report has been drawn up for all claims

| Place of search | Date of completion of the search | Examiner |
|---|---|---|
| Munich | 24 February 2006 | Hanisch, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 05 11 0145

This annex lists the patent family members relating to the patent documents cited in the above-mentioned European search report.
The members are as contained in the European Patent Office EDP file on
The European Patent Office is in no way liable for these particulars which are merely given for the purpose of information.

24-02-2006

| Patent document cited in search report | Publication date | Patent family member(s) | Publication date |
|---|---|---|---|
| US 6020205 A | 01-02-2000 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004068140 A

**Non-patent literature cited in the description**

- **GUO.** *J. Agric. Food Chem.,* vol. 45, 1787-1796